(19) Europäisches Patentamt
European Patent Office
Office européen des brevets

(11) Publication number: **0 130 041**
**B1**

(12) **EUROPEAN PATENT SPECIFICATION**

(45) Date of publication of patent specification: **13.01.88**

(51) Int. Cl.[4]: **C 07 C 93/14**, C 07 C 101/18, A 01 N 37/36, A 01 N 37/44

(21) Application number: **84304159.1**

(22) Date of filing: **20.06.84**

(54) **Novel herbicidal substituted pyridyl phenyl and diphenyl ethers, herbicidal compositions containing them, processes for the preparation thereof and the use thereof for combating weeds.**

(30) Priority: **23.06.83 US 507261**

(43) Date of publication of application:
**02.01.85 Bulletin 85/01**

(45) Publication of the grant of the patent:
**13.01.88 Bulletin 88/02**

(84) Designated Contracting States:
**BE CH DE FR GB IT LI LU NL SE**

(56) References cited:
**EP-A-0 072 348**
**EP-A-0 080 746**

(73) Proprietor: **Rohm and Haas Company**
**Independence Mall West**
**Philadelphia, Pennsylvania 19105 (US)**

(72) Inventor: **Beaulieu, Ann Hecht**
**1426 Vestra Road**
**Gwynedd, Pa. 19436 (US)**
Inventor: **Yih, Roy Yangming**
**94 Windover Lane**
**Doylestown, PA 18901 (US)**

(74) Representative: **Angell, David Whilton et al**
**ROHM AND HAAS (UK) LTD. European Operations Patent Department Lennig House**
**2 Mason's Avenue**
**Croydon CR9 3NB (GB)**

**EP 0 130 041 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention concerns the provision of new herbicidal substituted pyridyl phenyl and diphenyl ethers, the preparation thereof, herbicidal compositions containing them and methods of combating weeds.

Diphenyl ether herbicides containing an amino or substituted amino group are known.

U.S. Patent 4,039,588 discloses amino-substituted diphenyl ethers, such as 2,4-dichloro-3'-dimethylamino-4'-nitrodiphenyl ether and 2,4-dichloro-3'-isopropylamino-4'-nitrodiphenyl ether.

Japanese Patent Publication No. 72946, published May 7, 1982, discloses amino diphenyl ether derivatives, such as 2-chloro-4-trifluoromethyl-3'-amino-4' nitrodiphenyl ether, in which one of the two free nitrogen electrons participates in a covalent bond with a hydrogen atom, a lower alkyl group, a lower alkenyl group, or a lower alkynyl group, and the other free electron participates in a covalent bond with an alkoxycarbonylalkyl group.

German Patent 2960829, published March 12, 1981, discloses 2-chloro-6-nitro-3-(phenoxy or phenylthio) aniline plant growth regulants and herbicides, wherein one of the two free aniline nitrogen electrons is bonded to nitrogen, alkyl, cycloalkyl, chloro- or hydroxy-substituted alkyl, allyl or trifluoroacetyl, and the other free aniline nitrogen electron is bonded to hydrogen, methyl, ethyl, *n*-propyl, or isopropyl, or the two free aniline nitrogen electrons may be bonded to an alkylene group having up to five members, optionally interrupted by O, NH, $NCH_3$ or $CH—N(CH_3)_2$.

German Patent Application DE2938595, published April 23, 1981, discloses a process for producing 2- or 4-chloro-3-phenoxy-6-nitro-N-substituted anilines, wherein the N-substituent can be hydrogen, alkyl, alkenyl, alkynyl, hydroxy, alkoxy, phenoxy, halophenoxy, alkylphenoxy, alkoxyphenoxy, nitrophenoxy, cyanophenoxy, amino, alkylthio, benzyl, alkoxycarbonylalkyl, aminocarbonylalkyl, and the like.

German Patent Application DE2926829 discloses 2-chloro-4-trifluoromethylphenyl-3'-nitrophenyl ethers having in the 4'-position a hydrogen atom or a nitro group, and 2-chloro-4-trifluoromethylphenyl-4'-nitrophenyl ethers having in the 3'-position a nitro, alkoxy, alkylthio, hydroxy, sulfhydryl, amino, and monoalkylamino. U.S. Patent 4,277,624, issued July 7, 1981, corresponds to German Patent Application DE2926829.

European Patent Application 72348, published February 16, 1983, discloses, *inter alia*, herbicidal 2-chloro-4-trifluoromethyl-4'-nitrophenyl ethers having in the 3' position an amino group substituted with a group of the formula $(CHR^1)_mG$ in which $R^1$ is H or alkyl optionally substituted with hydroxy, alkyloxy or alkylthio, m is 2 to 5 and G is $—COO(C_1$ to $C_3)$alkyl or $—COS(C_1$ to $C_3)$alkyl in which the alkyl may be substituted by halogen or cyano, specifically the compound having in the 3' position the group $—N(H)—CH_2CH_2—COOCH_2Cl$.

In accordance with the present invention there is provided a new class of ethers having two aromatic groups attached to the ether oxygen atom and having a substituted amino group attached to a phenyl ring characterized in that the ethers are of the formula

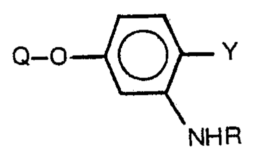

(I)

wherein:

Q is a group of the formula

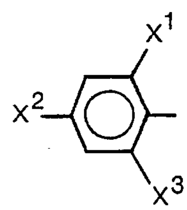

or

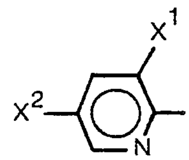

$X^1$ is a hydrogen atom, a cyano group, or a halogen atom, preferably a halogen atom and most preferably a chlorine atom;

$X^2$ is a haloalkyl group, preferably a trifluoromethyl group, or a halogen atom;

$X^3$ is a hydrogen atom or a halogen atom;

Y is a nitro group, a cyano group, or a halogen atom;

R is a group of the formula

$$—(CHR^1)_m—A—(CHR^2)_n—R^3$$

$R^1$ and $R^2$, independently of each other and in each $(CHR^1)$ and $(CHR^2)$ group, are a hydrogen atom; an unsubstituted or substituted $(C_1—C_6)$ alkyl group; a $(C_1—C_6)$ alkoxy group; or a $(C_1—C_6)$ alkylthio group; provided that $R^1$ and $R^2$ may not be alkoxy or alkylthio alpha to an —NH— moiety;

A is —$N(R^1)$—, —N(O)—, —O—, —S—, —S(O)—, —$S(O)_2$—, —C(O)—, —C(O)B—, or —$N(H)S(O)_2$—;

B is a ($C_1$—$C_6$) alkylene group, —O—, —S—, or —$N(R^4)$—;

$R^3$ is a cyano group, or a group of the formula —C(O)Z provided that when $R^3$ is cyano, B is a ($C_1$—$C_6$) alkylene group or $N(R^4)$;

$R^4$ is a hydrogen atom or an unsubstituted or substituted ($C_1$—$C_6$) alkyl group;

Z is a hydrogen atom; an unsubstituted or substituted ($C_1$—$C_6$) alkyl group; —$N(R^4)_2$; —$OR^5$; —$SR^5$; or —$N(R^4)SO_2R^6$ provided that when Z is hydrogen, A is not or —N(H)— or —$N(H)S(O)_2$—; B is not —N(H)— and $R^1$, $R^2$ or $R^4$ when being substituted allyl groups, comprise no amino or mono-($C_1$—$C_6$)alkylamino group;

$R^5$ is a hydrogen atom; an unsubstituted or substituted ($C_1$—$C_6$) alkyl group, or an agronomically-acceptable cation, preferably an alkali metal, alkaline earth metal, ammonium, substituted ammonium, sulfonium, substituted sulfonium, sulfoxonium, or substituted sulfoxonium cation;

$R^6$ is an unsubstituted ($C_1$—$C_6$) alkyl group, a hydroxy group or an agronomically-acceptable salts formed with such group, an unsubstituted or substituted phenyl group with up to three substitutnets which can be the same or different and are selected from chlorine, bromine, fluorine ($C_1$—$C_6$)alkoxy and ($C_1$—$C_6$) alkyl which is unsubstituted or substituted with up to three substituents which can be the same or different and are selected from chlorine, bromine, and ($C_1$—$C_6$) alkoxy;

m is an integer from 2 to 10; and

n is an integer from 1 to 3.

The ethers of Formula I above are distinguished from known compounds by possessing the characteristic group —NHR in conjunction with the other ring substituents present and this results in compounds possessing good selectivity and herbicidal activity. Usually, in the ethers of Formula I, the chain length between the phenyl-attached nitrogen atom in the group —NHR and the chain terminal group (cyano or C(O)Z) is from 4 to 14 atoms.

When, in Formula I, $R^1$, $R^2$, $R^4$, or $R^5$ is a substituted alkyl group, there can be up to three substituents which can be the same or different. The substituents will be selected from chlorine, bromine, fluorine, hydroxy, ($C_1$—$C_6$)alkoxy, ($C_1$—$C_6$) alkylthio, ($C_1$—$C_6$) alkylcarbonyl, ($C_1$—$C_6$)alkoxycarbonyl, amino, mono- or di-($C_1$—$C_6$) alkylamino, ($C_1$—$C_6$) alkylcarbonyloxy and unsubstituted or substituted phenyl, phenoxy, or phenylthio each of these aromatic groups having up to three substituents which can be the same or different and are selected from chlorine, bromine, fluorine, hydroxy, ($C_1$—$C_6$) alkoxy, ($C_1$—$C_6$) alkylthio and ($C_1$—$C_6$) alkyl which is unsubstituted or substituted with up to three substituents which can be the same or different and are selected from chlorine, bromine, fluorine, hydroxy and ($C_1$—$C_6$) alkoxy.

In the ethers of the invention aliphatic hydrocarbyl moieties in groups such as alkyl, alkoxy, alkylthio, alkylene etc. may be straight or branched chain. Halo substituents are typically bromo, chloro or fluoro.

Among the agronomically-acceptable salts are those in which the agronomically-acceptable cation is an alkali metal cation, such as sodium or potassium, or an alkaline earth metal cation, such as calcium, magnesium, barium, or strontium, an ammonium cation, such as those having the formula $NZ^1Z^2Z^3Z^4$, wherein each of $Z^1$, $Z^2$, $Z^3$, and $Z^4$ is individually a hydrogen atom, a hydroxy group, a ($C_1$—$C_4$) alkoxy group, a ($C_1$—$C_{20}$) alkyl group, a ($C_3$—$C_8$) alkenyl group, a ($C_3$—$C_8$) alkynyl group, a ($C_2$—$C_8$) hydroxyalkyl group, a ($C_2$—$C_8$) alkoxyalkyl group, a ($C_2$—$C_6$) aminoalkyl group, a ($C_2$—$C_6$) haloalkyl group, a substituted or unsubstituted phenylalkyl group, having up to 4 carbon atoms in the alkyl moiety, or any two of $Z^1$, $Z^2$, $Z^3$, or $Z^4$ can be taken together to form with the nitrogen atom a 5- or 6-member heterocyclic ring, optionally having up to one additional hetero oxygen, nitrogen, or sulfur atom in the ring, and preferably saturated, such as a piperidine, morpholino, pyrrolidino, or piperazino ring, or the like, or any three of $Z^1$, $Z^2$, $Z^3$ or $Z^4$ can be taken together to form with the nitrogen atom a 5- or 6-member aromatic heterocyclic ring, such as a piperazole or pyridine ring. When the ammonium group contains a substituted phenyl or substituted phenylalkyl group, the substituents will generally be selected from halogen atoms, ($C_1$—$C_8$) alkyl groups, ($C_1$—$C_4$) alkoxy groups, hydroxy groups, nitro groups, trifluoromethyl groups, cyano groups, amino groups and ($C_1$—$C_4$) alkylthio groups. Such substituted phenyl groups preferably have up to two such substituents. Representative ammonium cations include ammonium, dimethylammonium, 2-ethylhexylammonium, bis(2-hydroxyethyl)ammonium, tris(2-hydroxyethyl)ammonium, dicyclohexylammonium, t-octylammonium, 2-hydroxyethylammonium, morpholinium, piperidinium, 2-phenethylammonium, 2-methyl-benzylammonium, n-hexylammonium, triethylammonium, trimethylammonium, tri(n-butyl)ammonium, methoxyethylammonium, diisopropylammonium, pyridinium, diallylammonium, pyrazolium, propargylammonium, dimethylhydrazinium, hydroxyammonium, methoxyammonium, dodecylammonium, octadecylammonium, 4-dichlorphenylammonium,- 4-nitrobenzylammonium, benzyltrimethyl-ammonium, 2-hydroxyethyldimethyloctadecylammonium, 2-hydroxyethyldiethyloctyl-ammonium, decyltrimethylammonium, hexyltriethylammonium and 4-methylbenzyltrimethylammonium.

Preferably, the compounds of the invention are those of the formula

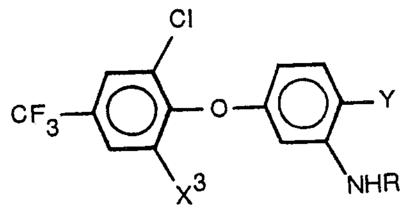

(Ia)

wherein

R is as defined above;

$X^3$ is a hydrogen atom, a chlorine atom, or a fluorine atom, most preferably a hydrogen atom;

Y is a nitro group, a chlorine atom, or a bromine atom;

$R^1$ and $R^2$, independently of each other and in each ($CHR^1$) and ($CHR^2$) group, are a hydrogen atom or an unsubstituted or substituted ($C_1$—$C_6$) alkyl group;

A is —N($R^1$)— wherein $R^1$ is a hydrogen atom or an unsubstituted or substituted ($C_1$—$C_6$) alkyl group, —O—, —S—, —C(O)—, or —C(O)B—;

B is —O—, —N($R^4$)—, or a ($C_1$—$C_6$) alkylene group;

$R^3$ is as defined above;

$R^4$ is a hydrogen atom or an unsubstituted or subsituted ($C_1$—$C_6$) alkyl group;

Z is —$OR^5$ or —N($R^4$)$SO_2R^5$;

$R^5$ is a hydrogen atom, an unsubstituted or substituted ($C_1$—$C_6$) alkyl group, or an agronomically-acceptable alkaline earth metal cation, an alkali metal cation or an ammonium or substituted ammonium cation;

$R^6$ is an unsubstituted ($C_1$—$C_6$) alkyl group;

m is 2 or 3; and

n is 1.

More preferably, the compounds are those of Formula (Ia) wherein

$X^3$ is a hydrogen atom;

Y is a nitro group, a chlorine atom, or a bromine atom;

$R^1$ and $R^2$, independently of each other and in each ($CHR^1$) and ($CHR^2$) group, are a hydrogen atom or an unsubstituted or substituted ($C_1$—$C_6$) alkyl group;

A is —O—, —N(H)—, —N($CH_3$)—, —N($CH_2OCH_3$)—, —S—, —C(O)—, or —C(O)B—;

B is —O— or a ($C_1$—$C_6$) alkylene group;

$R^3$ is as defined above;

Z is —$OR^5$;

$R^5$ is an unsubstituted or substituted ($C_1$—$C_6$) alkyl group, or an alkaline earth metal, an alkali metal, or ammonium or substituted ammonium cation;

m is 2 or 3; and

n is 1.

Still more preferably, the compounds are those of Formula (Ia) wherein

$X^3$ is a hydrogen atom;

Y is a nitro group, a chlorine atom, or a bromine atom;

$R^1$ and $R^2$, independently of each other and in each ($CHR^1$) and ($CHR^2$) group, are a hydrogen atom or an unsubstituted or substituted ($C_1$—$C_6$) alkyl group;

A is —O—, —N(H)—, —S—, —C(O)—, or —C(O)B—;

B is —O—;

$R^3$ is as defined above;

Z is —$OR^5$;

$R^5$ is a hydrogen atom, an unsubstituted or substituted ($C_1$—$C_6$) alkyl group, or an alkaline earth metal, an alkali metal, or substituted ammonium cation;

m is 2 or 3; and

n is 1.

Most preferably, the compounds are those of Formula (Ia) wherein

$X^3$ is a hydrogen atom;

Y is a nitro group or a chlorine atom;

$R^1$ and $R^2$, independently of each other and in each ($CHR^1$) and ($CHR^2$) group, are a hydrogen atom or an unsubstituted or substituted ($C_1$—$C_6$) alkyl group;

A is —O—, —N(H)—, —S—, —C(O)—, or —C(O)B—;

B is —O—;

$R^3$ is as defined above;

Z is —$OR^5$;

$R^5$ is a hydrogen atom; an unsubstituted or substituted ($C_1$—$C_6$) alkyl group, or an alkali metal cation, preferably sodium or potassium, or an ammonium or substituted ammonium cation;

m is 2 or 3; and

n is 1.

The compounds of the present invention can be made by a wide variety of conventional reaction procedures. For example, compounds of the present invention in which Y is a nitro group can be prepared by the following general procedure. To a solution of *m*-nitrophenol in a polar solvent, such as dimethylsulfoxide, dimethylformamide, sulfolane, dioxane, is added a base, such as potassium hydroxide, potassium carbonate, triethylamine, followed by a compound of the formula Q—Cl, wherein Q is as defined above. The mixture is allowed to stir at any temperature between about 25° and about 220°, depending on the solvent, preferably at about 100—180°. Upon completion, the reaction mixture is diluted with water, extracted with an organic solvent, such as ether, methylene chloride, hexane, ethyl acetate, ethylene dichloride. The volatiles are removed under reduced pressure and the crude product is purified by distillation. The mononitro product above is dissolved in an organic solvent, such as methylene chloride, ethylene dichloride, and stirred vigorously with concentrated sulfuric acid. Potassium nitrate is added such that the internal temperature of the reaction is maintained between about −10 to 10°C. Upon completion of the reaction the product is purified as above and the final product was recrystallized from an appropriate solvent, such as ethanol, to yield a compound of the formula

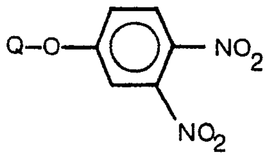

(II)

wherein Q is as defined above.

An amine of the formula $NH_2R$, wherein R is as defined above, is allowed to react with the compound of formula (II) in a polar solvent, such as dimethylsulfoxide, dioxane, dimethylformamide, sulfolane, ethanol, tetrahydrofuran, in the presence of base, such as the following potassium salts: carbonate, hydroxide, t-butoxide, or nitrogen bases, such as triethylamine or diisopropyl ethylamine, at between about −24 and 150°C, preferably 25—100°C. Upon completion, products purified as above and the final product were either recrystallized from the appropriate polar solvent or purified by column chromatography, to yield the desired ether of the invention having the formula

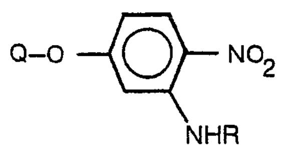

(III)

Free acids can be esterified using one of the following procedures: acid catalysts, such as hydrochloric acid or p-toluenesulfonic acid in an alcohol, such as methanol, ethanol, or isopropanol; treatment with base, such as potassium hydroxide, carbonate or hydride followed by an alkylating agent, such as iodomethane, isopropyl bromide; treatment with a chloroformate, such as methyl or ethyl chloroformate in the presence of an organic base, such as triethylamine or pyridine in an organic solvent, such as benzene, toluene.

In another procedure, when A is —O—, a compound of the formula

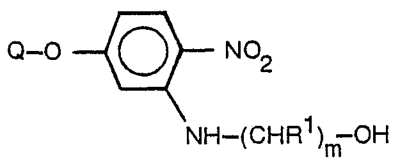

(IV)

wherein Q, $R^1$ and m are as defined above, and which has been prepared by the procedure described above with an amine of the formula $NH_2—(CHR^1)_m—OH$, is dissolved in a suitable inert solvent and the solution is added to a suspension or solution of a suitable, conventional base, such as, for example, sodium hydride or hydroxide or alkoxide at temperatures ranging from about −10°C. to about 120°C., examples of said suitable inert solvent being tetrahydrofuran, dimethoxyethane, dimethylsulfoxide, and sulfolane, preferably tetrahydrofuran. An alkylating agent of the formula $X—(CHR^2)_n—R^3$, wherein X is a halogen atom, preferably a bromine atom or other leaving group, and $R^2$, $R^3$ and n are as defined above, is added and the resulting mixture is stirred at a temperature of from about room temperature to reflux temperature. The reaction mixture is then diluted with water, extracted with an organic solvent, and the extracts are dried over a drying agent, such as, for example, magnesium sulfate, potassium carbonate, sodium sulfate, and molecular sieves. Filtration of the drying agent and concentration of the organic filtrate by evaporating the solvent under reduced pressure affords the desired product.

Compounds of the present invention in which Y is a cyano group or a halogen atom can be prepared, for example, by the following general procedure. Following the procedure set forth above, a compound of the formula Q—Cl, wherein Q is as defined above, is reacted with a compound of the formula

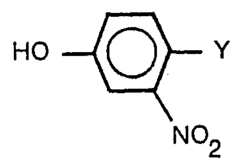
(V)

wherein Y is a cyano group or a halogen atom. The final product is then reduced using conventional techniques, for example, catalytic hydrogenation using a palladium or platinum or charcoal catalyst and a polar solvent, such as ethyl acetate, acetic acid or ethanol, and purified by column chromatography, to give a compound of the formula

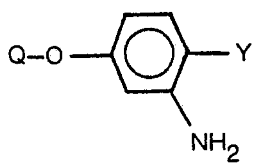
(VI)

wherein Q is as defined above and Y is a cyano group or a halogen atom.

The compound of formula (VI) is allowed to react with an appropriately-substituted electrophile, for example, a compound having the formula XR, wherein X is a halogen atom, preferably a bromine atom, or other leaving group, in a polar solvent in the presence of base. Upon completion of the reaction, the mixture is diluted with water, extracted with an organic solvent, such as ether, methylene chloride, ethyl acetate, and concentrated under reduced pressure to afford the crude product, which can be purified by column chromotography.

Compounds of the invention can also be made by appropriate post-reactions involving intermediates prepared by the procedures set forth above or by other conventional preparative techniques.

Typical compounds representative of the present invention include the following:

Methyl, ethyl, and sodium 2-(3-N-(5-(2-chloro-4-(trifluoromethyl)phenoxy)-2-nitrophenyl)aminopropoxy)acetate

Methyl, ethyl, and sodium 2-(2-N-(5-(2-chloro-4-(trifluoromethyl)phenoxy)-2-nitrophenyl)aminoethoxy)acetate

Methyl, ethyl, and sodium 2-(2-N-(5-(2-chloro-4-(trifluoromethyl)phenoxy)-2-nitrophenyl)aminoethoxy)propionate

Methyl, ethyl, and sodium 2-(1-N-(5-(2-chloro-4-(trifluoromethyl)phenoxy)-2-nitrophenyl)aminoethoxy)acetate

Methyl, ethyl, and sodium 2-(2-N-(5-(2-chloro-4-(trifluoromethyl)phenoxy)-2-nitrophenyl)aminopropoxy)acetate

Methyl, ethyl, and sodium 2-(2-N-(5-(2-chloro-4-(trifluoromethyl)phenoxy)-2-nitrophenyl)aminoisopropoxy)acetate

Methyl, ethyl, and sodium 2-(2-N-(5-(2-chloro-4-(trifluoromethyl)phenoxy)-2-nitrophenyl)aminoethylamino)acetate

Methyl, ethyl, and sodium 2-(2-N-(5-(2-chloro-4-(trifluoromethyl)phenoxy)-2-nitrophenyl)aminothioisopropoxy)acetate

Methoxy carbonyl-, ethoxy carbonyl-, and sodium carboxymethyl-3-N-(5-(2-chloro-4-(trifluoromethyl)phenoxy)-2-nitrophenyl)amino)propionate

1-(Methoxycarbonyl)-4-N-(5-(2-chloro-4-(trifluoromethyl)phenoxy)-2-nitrophenyl)amino)but-2-none, and corresponding ethyl ester and sodium salt

1-(Thiomethoxycarbonyl)-4-N-(5-(2-chloro-4-(trifluoromethyl)phenoxy)-2-nitrophenyl)amino)but-2-none, and corresponding ethyl ester and sodium salt

2-(2-N-(5-(2-chloro-4-(trifluoromethyl)phenoxy)-2-nitrophenyl)aminothiopropoxy)acetonitrile

3-(3-N-(5-2-chloro-4-(trifluoromethyl)phenoxy)-2-nitrophenyl)aminopropoxy)propionitrile

3-(1-ethyl-3-N-(5-(2-chloro-4-(trifluoromethyl)phenoxy)-2-nitrophenyl)aminopropylamino)propionitrile

Methyl, ethyl, and sodium 2-(1-methoxy-2-N-(5-(2-chloro-4-(trifluoromethyl)phenoxy)-2-nitrophenyl)aminoethoxy)acetate

Methyl, ethyl, and sodium 2-(2-methoxy methyl)-2-N-(5-(2-chloro-4-(trifluoromethyl)phenoxy)-2-nitrophenyl)aminoethoxy)acetate

Methoxymethyl 2-(2-N-(5-(2-chloro-4-(trifluoromethyl)phenoxy)-2-nitrophenyl)aminoethoxy)acetate, and corresponding ethyl ester and sodium salt

Methyl, ethyl, and sodium 2-(2-N-(5-(2-chloro-4-(trifluoromethyl)phenoxy)-2-bromophenyl)aminoethoxy)acetate

Methyl, ethyl, and sodium 2-(2-N-(5-(2-chloro-4-(trifluoromethyl)phenoxy)-2-chlorophenyl)aminoethoxy)propionate
Methyl, ethyl, and sodium 2-(2-N-(5-(2,6-dichloro-4-(trifluoromethyl)phenoxy)-2-bromophenyl)aminoethoxy)acetate
Methyl, ethyl, and sodium 2-(2-N-(5-(2-chloro-4-(trifluoromethyl)phenoxy)-2-chlorophenyl)aminoisopropoxy)acetate
Methyl, ethyl, and sodium 2-(2-N-(5-(2,4-dichloro phenoxy)-2-nitrophenyl)aminoethoxy)acetate
Methyl, ethyl, and sodium 2-(2-N-(5-2-chloro-4-bromophenoxy)-2-nitrophenyl)aminoethoxy)propionate
Methyl, ethyl, and sodium 2-(2-N-(5-2-cyano-4-(trifluoromethyl)phenoxy)-2-nitrophenyl)aminoethoxy)acetate
Methyl, ethyl, and sodium 2-(2-N-(5-(2-chloro-4-cyanophenoxy)-2-nitrophenyl)aminopropoxy)acetate
Methyl, ethyl, and sodium 2-(2-N-(5-2,4-dichloro)-phenoxy)-2-bromophenyl)aminoisopropoxy)acetate
Propyl, isopropyl, butyl, *sec*-butyl, *tert*-butyl, pentyl, and hexyl 2-(2-N-(5-(2-chloro-4-(trifluoromethyl)phenoxy)-2-chlorophenyl)aminoethoxy)propionate
Methyl, ethyl, and sodium 2-(2-N-(5-(3-chloro-5-(trifluoromethyl)pyridyloxy)-2-nitrophenyl)aminoethoxy)acetate
Methyl, ethyl, and sodium 2-(2-N-(5-(3-chloro-5-(trifluoromethyl)pyridyloxy)-2-nitrophenyl)aminopropoxy)acetate
Methyl, ethyl, and sodium 2-(2-N-(5-(2-cyano-4-(trifluoromethyl)phenoxy)-2-nitrophenyl)aminoethoxy)acetate
Propyl, isopropyl, butyl, *sec*-butyl, tert-butyl, isobutyl, pentyl, and hexyl 2-(2-N-(5-(2-chloro-4-(trifluoromethyl)phenoxy)-2-nitrophenyl)aminoethoxy)acetate
Methyl, ethyl, and sodium 2-(2-N-(5-(2-chloro-4-(difluoroethyl)phenoxy)-2-nitrophenyl)aminoethoxy)acetate
Methyl, ethyl, and sodium 2-(2-N-(5-(2-chloro-4-(difluorochloromethyl)phenoxy)-2-nitrophenyl)aminoethoxy)acetate
Methyl, ethyl, and sodium 2-(2-N-(5-(2,6-dichloro-4-(trifluoromethyl)phenoxy)-2-nitrophenyl)aminoethoxy)acetate
as well as the corresponding free acids of each of the foregoing compounds.

Preferred compounds of the present invention include the following:
1-(Methoxycarbonyl)-4-N-(5-(2-chloro-4-(trifluoromethyl)phenoxy)-2-nitrophenyl)amino)but-2-one
Methyl 2-(2-N-(5-(2,4-dichloro phenoxy)-2-nitrophenyl)aminoethoxy)acetate
Methyl 2-(2-N-(5-(2-chloro-4-(trifluoromethyl)phenoxy)-2-chlorophenyl)aminoethoxy)propionate
Methyl 1-(2-N-(5-(2-chloro-4-(trifluoromethyl)phenoxy)-2-bromophenyl)aminoethoxy)acetate
Methyl 2-(2-N-(5-(2-chloro-4-(trifluoromethyl)phenoxy)-2-cyanophenyl)aminopropoxy)acetate
Methyl 2-(2-N-(5-(2-chloro-4-(trifluoromethyl)phenoxy)-2-nitrophenyl)aminoethoxy)acetate
Methyl 2-(2-N-(5-(2-chloro-4-(trifluoromethyl)phenoxy)-2-nitrophenyl)aminopropoxy)acetate
Methyl 2-(2-N-(5-(2-chloro-4-(trifluoromethyl)phenoxy)-2-nitrophenyl)aminoisopropoxy)acetate
Methyl 2-(3-N-(5-(2-chloro-4-(trifluoromethyl)phenoxy)-2-nitrophenyl)aminopropoxy)acetate

The ethyl esters and sodium salts of the above compounds, as well as the corresponding acids, are also among the preferred compounds of the present invention.

The most preferred compounds of the present invention include the following:
Methyl 2-(2-N-(5-(2-chloro-4-(trifluoromethyl)phenoxy)-2-nitrophenyl)aminoethoxy)acetate
Methyl 2-(2-N-(5-(2-chloro-4-(trifluoromethyl)phenoxy)-2-nitrophenyl)aminopropoxy)acetate
Methyl 2-(2-N-(5-(2-chloro-4-(trifluoromethyl)phenoxy)-2-nitrophenyl)aminoisopropoxy)acetate
Methyl 2-(3-N-(5-(2-chloro-4-(trifluoromethyl)phenoxy)-2-nitrophenyl)aminopropoxy)acetate
Methyl 2-(3-N-(5-(2-chloro-4-(trifluoromethyl)phenoxy)-2-nitrophenyl)aminobutoxy)acetate
Methyl 2-(3-N-(5-(2-chloro-4-(trifluoromethyl)phenoxy)-2-nitrophenyl)aminobutoxy)propionate

Further examples of compounds of the present invention (some of which are also named above) include those of formula (Ia) in which $X^3$ is a hydrogen atom, Y is a nitro group or a chlorine atom, and NHR is
$NHCH_2CH_2CH_2OCH_2CO_2CH_3$
$NHCH(CH_3)CH_2CH_2OCH_2CO_2CH_{32}$
$NHCH_2CH_2CH_2CH_2OCH_2CO_2CH_3$
$NHCH(CH_3)CH_2CH_2CH_2OCH_2CO_2CH_3$
$NHCH_2CH_2CH_2CH_2CH_2OCH_2CO_2CH_3$
$NHCH(CH_3)CH_2CH_2CH_2CH_2OCO_2CH_3$
$NHCH_2CH_2OCH_2CH_2OCH_2CO_2CH_3$

$NHCH_2CH_2SCH_2CO_2CH_3$
$NHCH(CH_3)CH_2SCH_2CO_2CH_3$
$NHCH_2CH_2CH_2SCH_2CO_2CH_3$
$NHCH(CH_3)CH_2CH_2SCH_2CO_2CH_3$
$NHCH_2CH_2CH_2CH_2SCH_2CO_2CH_3$
$NHCH(CH_3)CH_2CH_2CH_2SCH_2CO_2CH_3$

$NHCH_2CH_2NHCH_2CO_2CH_3$
$NHCH_2CH_2NHCH_2CH_2CO_2CH_3$
$NHCH_2CH_2N(CH_3)CH_2CO_2CH_3$
$NHCH_2CH_2N(CH_2OCH_3)CH_2CO_2CH_3$
$NHCH(CH_3)CH_2NHCH_2CO_2CH_3$
$NHCH_2CH_2CH_2NHCH_2CO_2CH_3$
$NHCH(CH_3)CH_2CH_2NHCH_2CO_2CH_3$
$NHCH_2CH_2NHSO_2CH_2CO_2CH_3$
$NHCH_2CH_2N(CH_3)SO_2CH_2CO_2CH_3$
$NHCH_2CH_2COCH_2CO_2CH_3$
$NHCH_2(CH_3)CH_2COCH_2CO_2CH_3$

as well as the isomers of the above compounds in which the position of the side-chain methyl group is varied, compounds corresponding to the above compounds in which —S— is replaced by —S(O)— or by —$S(O)_2$— and the ethyl esters, sodium salts, and free acids of each of the above compounds.

The novel ethers of the invention are useful both as preemergence and as postemergence herbicides. Preemergence herbicides are ordinarily used to treat the soil in which the desired crop is to be planted by application either before seeding or planting, during seeding or planting, or, as in most applications, after seeding and before the crop emerges. Postemergence herbicides are those which are applied after the weed plants have emerged and during their growth period.

Among the crops on which ethers of the invention can be advantageously employed are, for example, cotton, soybeans, peanuts, safflower, beans, rice, peas, carrots, maize, wheat and other cereal crops.

The ethers of the invention can be applied in any amount which will give the required control of weeds. A preferred rate of application of the herbicides of the invention is from 0.01 to about 13.5, and most preferably about 0.1 to about 4.5 kg of the ether per hectare.

Under some conditions, the ethers of the invention may be advantageously incorporated into the soil or other growth medium prior to planting a crop. This incorporation can be carried out by any convenient means, including by simple mixing with the soil, by applying the ether to the surface of the soil and then discing or dragging into the soil to the desired depth, or by employing a liquid carrier to accomplish the necessary penetration and impregnation.

When used in transplanted rice crops, ethers of the invention can be applied either preemergence or postemergence to the weeds — that is, they can be applied to the growth medium of the transplanted plants either before the weed plants have emerged or while they are in their early stages of growth. The ethers can be applied to the growth medium either before or after the rice has been transplanted to that medium.

An ether of the invention can be applied to the growth medium or to plants to be treated either by itself or, as is generally done, as a component in a herbicidal composition or formulation which also comprises an agronomically-acceptable carrier or diluent. By agronomically-acceptable carrier or diluent is meant any substance which can be used to dissolve, disperse, or diffuse a herbicidal compound in the composition without impairing the effectiveness of the herbicidal compound and which by itself or when diluted, e.g. with water, has no detrimental effect on the soil, equipment, crops, or agronomic environment. Mixtures of the ethers of the invention may also be used in any of these herbicidal formulations which can also contain one or more other herbicides known in the art and which are compatible. The herbicidal compositions of the invention can be either solid or liquid formulations or solutions. For example, diphenyl ethers of the invention can be formulated as solutions, wettable powders, emulsifiable concentrates, dusts, granular formulations, aerosols, or flowable emulsion concentrates. In such formulations, the compounds are extended with a liquid or solid carrier and, when desired, suitable surfactants are incorporated.

Postemergence applications, to include adjuvants, such as surfactants, wetting agents, spreading agents, dispersing agents, stickers, adhesives, and the like, in accordance with agricultural practices.

The ethers of the invention may be formulated by following the general directions, modified if necessary having regard to the chemical and physical properties of the ethers of the invention, given in European patent application No. 20052A, Australian patent application No. 80/58498, Israeli patent application No. 60075, New Zealand patent application No. 193704 and South African patent No. 80/2938. The patent specifications just mentioned also contain examples of other herbicides which may be used in conjunction, usually in admixture, with the ethers of the invention.

The following compounds, coupled with their mode of preparation are illustrative of the present invention. The following Compound Nos. 1—6 are represented by Formula (Ib) below.

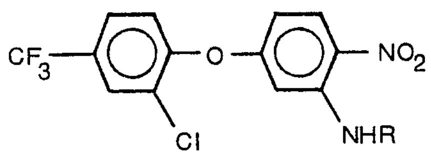

(Ib)

Compound No. 1
Methyl 2-(2-N-(5-2-chloro-4-(trifluoromethyl)phenoxy)-2-nitrophenyl)aminoethoxy)acetate

Compound No. 2
Methyl 2-(2-N-(5-(2-chloro-4-(trifluoromethyl)phenoxy)-2-nitrophenyl)aminoethoxy)propionate

Compound No. 3
Methoxycarbonylmethyl 3-N-(5-(2-chloro-4-(trifluoromethyl)phenoxy)-2-nitrophenyl)amino)propionate

Compound No. 4
Methyl 2-(2-N-(5-(2-chloro-4-(trifluoromethyl)phenoxy)-2-nitrophenyl)aminoisopropoxy)acetate

Compound No. 5
Methyl 2-(2-N-(5-(2-chloro-4-(trifluoromethyl)phenoxy)-2-nitrophenyl)aminopropoxy)acetate

Compound No. 6
Isopropyl 2-(2-N-(5-(2-chloro-4-(trifluoromethyl)phenoxy)-2-nitrophenyl)aminoisopropoxy)acetate

Preparation of Compound No. 1

To a suspension of sodium hydride (1.2 equivalents) in tetrahydrofuran at 10°C was added a solution of 2-N-(5-(2-chloro-4-(trifluoromethyl)phenoxy)-2-nitrophenyl)aminoethanol (1 equivalent). After the evolution of hydrogen ceased, methyl bromoacetate (two equivalents) was added. The final mixture was concentrated under reduced pressure, dissolved in ether, shaken with brine and dried over magnesium sulfate. Filtration and evaporation yielded Compound No. 1, mp 77—78°C.

By following substantially the same procedure set forth in the description of the preparation of Compound No. 1, except for replacing the methyl bromoacetate with methyl bromopropionate, Compound No. 2 was produced. Compound No. 3 was produced by alkylating (3-N-(5-(2-chloro-4-(trifluoromethyl)phenoxy)-2-nitrophenyl)aminopropionic acid with methyl bromoacetate using conventional alkylating reaction procedures.

Using a procedure analogous for the preparation of Compound No. 1, Compounds No. 4 and 6 were prepared by alkylating 1-N-(5-(2-chloro-4-(trifluoromethyl)phenoxy)-2-nitrophenyl)aminopropan-2-ol with methyl bromoacetate and separating Compounds 4 and 6 by high pressure liquid chromatography; and Compound 5 was prepared from alkylating 2-N-(5-(2-chloro-4-(trifluoromethyl)phenoxy-2-nitrophenyl)aminopropan-1-ol with methyl bromoacetate.

The nmr data for Compound Nos. 1—6 are listed in TABLE 1 and the elemental analytical data for Compound Nos. 1 and 3 are listed in TABLE 2 as follows:

TABLE 1

| Compound No. | R | NMR |
|---|---|---|
| 1 | $CH_2CH_2OCH_2CO_2CH_3$ | 8.7—6.1 (m,7); 4.2 (s, 2); 3.9 (m,5); 3.5 (m,2) |
| 2 | $CH_2CH_2OCH(CH_3)CO_2CH_3$ | 8.7—6.1 (m,7); 3.8 (m,8); 1.5 (d,3) |
| 3 | $CH_2CH_2CO_2CH_2CO_2CH_3$ | 8.7—6.2 (m,7); 4.7 (s, 2); 3.8 (m,5); 2.9 (t,2) |
| 4 | $CH_2CH(CH_3)OCH_2CO_2CH_3$ | 8.6 (br.m, 1H); 8.5—6.1 (m,6); 4.2 (s,2); 4.1—3.2 (m,6); 1.3 (d,3) |
| 5 | $CH(CH_3)CH_2OCH_2CO_2CH_3$ | 8.6—6.2 (m,7H); 4.4—3.4 (m,8H); 1.2 (d,3H) |
| 6 | $CH_2CH(CH_3)OCH_2CO_2iC_3H_7$ | 8.6 (br.t,1H); 8.4—6.2 (m,6); 5.2 (m,1); 4.2—3.8 (m,3); 3.3 (br.t.2H); 1.4 (d,9) |

TABLE 2

| Compound No. | Melting Point (°C) | Composition | Element | Calculated | found |
|---|---|---|---|---|---|
| 1 | 77—78° | $C_{18}H_{16}ClF_3N_2O_6$ | C | 48.2 | 47.99 |
| | | | H | 3.6 | 3.53 |
| | | | N | 6.2 | 6.17 |
| | | | Cl | 7.9 | 8.01 |
| | | | F | 12.7 | 12.45 |
| 3 | 60—61° | $C_{19}H_{16}ClF_3N_2O_7$ | C | 47.8 | 48.15 |
| | | | H | 3.4 | 4.05 |
| | | | N | 5.9 | 5.70 |
| | | | Cl | 7.4 | 6.68 |
| | | | F | 12.0 | 10.43 |

Listed in TABLE 3 are the results of the primary herbicide screen for the six diphenylethers in TABLE 1. The scale is based on 0 to 100; 0 = no control, 100 = 100% control (complete kill).

The following test procedure was employed. Seeds of selected crops and weeds were planted in soil in flats. For preemergence tests, the flats were treated with the test compound immediately after the planting. For postemergence tests, the seeds were allowed to germinate, and after about two weeks the flats are treated with the test compound. The compound to be evaluated was dissolved in a 50:50 by volume mixture of acetone and methanol containing a small amount (about 0.5 to 1.0 (w/v) %) surfactant, diluted with acetone, water or an acetone/water mixture and sprayed over the flats using a carrier volume equivalent to 4681/ha (50 U.S. gallons per acre) at the rate of application (kg/ha) specified in the tables. About two weeks after the application of the test compound, the state of growth of the plants was observed and the phytotoxic effect of the compound was evaluated.

The following abbreviations are used in TABLE 3:

| | |
|---|---|
| PRE = preemergence | |
| POST = postemergence | |
| CKL = cocklebur | *Xanthium pensylvanicum* |
| MA = marigold | *Tagetes spp.* |
| MG = morningglory | *Ipomoea spp.* |
| SIC = sicklepod | *Cassia obtusifolia* |
| TOM = tomato | *Lycopersicon esculentum* |
| VEL = velvetleaf | *Abutilon theophrasti* |
| BYG = barnyardgrass | *Echinochloa crus-galli* |
| DB = downy brome | *Bromus techorum* |
| FOX = foxtail | *Setaria viridis* |
| JON = Johnsongrass | *Sorghum halepense* |
| NUT = nutsedge | *Cyperus esculentus* |
| WO = wild oats | *Avena fatua* |

TABLE 3

| Compound No. | Rate kg/ha | Type Rest | CKL | MA | MG | SIC | TOM | VEL | BYG | DB | FOX | JON | NUT | WO |
|---|---|---|---|---|---|---|---|---|---|---|---|---|---|---|
| 1 | 2.242 | PRE | 100 | 100 | 100 | 100 | 100 | 100 | 77 | 100 | 100 | 95 | 10 | 60 |
| | 2.242 | POST | 100 | 100 | 100 | 100 | 100 | 100 | 28 | 15 | 65 | 35 | 10 | 25 |
| | .56 | PRE | 85 | 100 | 100 | 100 | 100 | 100 | 30 | 35 | 100 | 15 | 0 | 10 |
| | .56 | POST | 100 | 100 | 100 | 100 | 100 | 100 | 23 | 10 | 65 | 20 | 5 | 20 |
| 2 | .56 | PRE | 82 | 100 | 90 | 100 | 100 | 100 | 41 | 15 | 100 | 5 | 100 | 15 |
| | .56 | POST | 93 | 100 | 65 | 95 | 100 | 100 | 14 | 20 | 0 | 15 | 15 | 20 |
| | .112 | PRE | 14 | 10 | 10 | 0 | 10 | 55 | 6 | 0 | 25 | 0 | 10 | 0 |
| | .112 | POST | 81 | 100 | 55 | 55 | 100 | 90 | 8 | 15 | 0 | 10 | 0 | 15 |
| 3 | 2.242 | PRE | 83 | 100 | 100 | 80 | 100 | 100 | 39 | 45 | 100 | 15 | 0 | 10 |
| | 2.242 | POST | 98 | 100 | 100 | 90 | 100 | 100 | 17 | 25 | 30 | 15 | 10 | 5 |
| | .56 | PRE | 68 | 100 | 100 | 10 | 100 | 100 | 24 | 35 | 95 | 0 | 0 | 5 |
| | .56 | POST | 94 | 100 | 100 | 65 | 100 | 100 | 8 | 0 | 15 | 10 | 5 | 5 |
| 4 | .56 | PRE | 95 | —* | 80 | 90 | — | 100 | 0 | — | 100 | 0 | 0 | 0 |
| | .56 | POST | 100 | — | 100 | 100 | — | 100 | 15 | — | 90 | 45 | 20 | 45 |
| 5 | .224 | PRE | 100 | 100 | 100 | — | 100 | 100 | 98 | 100 | 100 | 90 | 0 | 90 |
| | .224 | POST | 100 | 100 | 100 | 100 | 100 | 100 | 55 | 65 | 100 | 20 | 10 | 15 |
| | .56 | PRE | 15 | 100 | 100 | — | 100 | 100 | 60 | 25 | 100 | 40 | 0 | 10 |
| | .56 | POST | 100 | 100 | 100 | 100 | 100 | 100 | 25 | 45 | 60 | 10 | 5 | 10 |
| 6 | .56 | PRE | 76 | — | 100 | 95 | — | 100 | 10 | — | 100 | 10 | 100 | 0 |
| | .56 | POST | 100 | — | 100 | 100 | — | 100 | 20 | — | 60 | 20 | — | 35 |

**Claims**

1. An ether having two aromatic groups attached to the ether oxygen atom and having a substituted amino group attached to a phenyl ring, characterised in that the ether is of the formula:

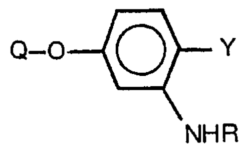

(I)

wherein

Q is a group of the formula

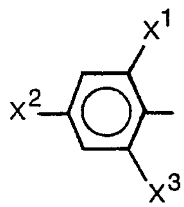 or 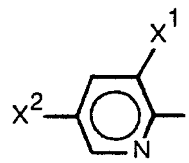

$X^1$ is a hydrogen atom, a cyano group, or a halogen atom;

$X^2$ is a haloalkyl group or a halogen atom,

$X^3$ is a hydrogen atom or a halogen atom;

Y is a nitro group, a cyano group, or a halogen atom;

R is a group of the formula

$$-(CHR^1)_m-A-(CHR^2)_n-R^3;$$

$R^1$ and $R^2$, independently of each other and in each $(CHR^1)$ and $(CHR^2)$ group, are a hydrogen atom, an unsubstituted or substituted $(C_1-C_6)$ alkyl group, a $(C_1-C_6)$ alkoxy group, or a $(C_1-C_6)$ alkylthio group, provided that $R^1$ and $R^2$ may not be alkoxy or alkylthio alpha to an —NH— moiety;

A is $-N(R^1)-$, $-N(O)-$, $-O-$, $-S-$, $-S(O)-$, $-S(O)_2-$, $-C(O)-$, $-C(O)B-$, or $-N(H)S(O)_2-$;

B is a $(C_1-C_6)$ alkylene group, $-O-$, $-S-$, or $-N(R^4)-$;

$R^3$ is a cyano group, or a group of the formula $-C(O)Z$ provided that when $R^3$ is cyano, B is a $C_1-C_6$ alkylene group or $N(R^4)$;

$R^4$ is a hydrogen atom or an unsubstituted or substituted $(C_1-C_6)$ alkyl group;

Z is a hydrogen atom; an unsubstituted or substituted $(C_1-C_6)$ alkyl group; $-N(R^4)_2$; $-OR^5$; $-SR^5$; or $-N(R^4)SO_2R^6$ provided that when Z is hydrogen, A is not $-N(H)-$ or $-N(H)S(O)_2-$; B is not $-N(H)$ and $R^1$, $R^2$ or $R^4$ when being substituted allyl groups, comprise no amino or mono-$(C_1-C_6)$alkylamino group;

$R^5$ is a hydrogen atom; an unsubstituted or substituted $(C_1-C_6)$ alkyl group, or an agronomically-acceptable cation;

$R^6$ is an unsubstituted $(C_1-C_6)$ alkyl group, a hydroxy group or an agronomically-acceptable salts formed with such group, an unsubstituted or substituted phenyl group with up to three substituents which can be the same or different and are selected from chlorine, bromine, fluorine $(C_1-C_6)$alkoxy and $(C_1-C_6)$ alkyl which alkyl is unsubstituted or substituted with up to three substituents which can be the same or different and are selected from chlorine, bromine, and $(C_1-C_6)$ alkoxy;

m is an integer from 2 to 10; and

n is an integer from 1 to 3;

and wherein

when $R^1$, $R^2$, $R^4$, or $R^5$ is a substituted alkyl group, there can be up to three substituents which can be the same or different and are selected from chlorine, bromine, fluorine; hydroxy, $(C_1-C_6)$ alkoxy, $(C_1-C_6)$ alkylthio, $(C_1-C_6)$ alkylcarbonyl, $(C_1-C_6)$ alkoxycarbonyl, amino, mono- or di-$(C_1-C_6)$ alkylamino, $(C_1-C_6)$ alkylcarbonyloxy and unsubstituted or substituted phenyl, phenoxy, or phenylthio each of these aromatic groups having up to three substituents which can be the same or different and are selected from chlorine, bromine, fluorine, hydroxy, $(C_1-C_6)$ alkoxy, $(C_1-C_6)$ alkylthio and $(C_1-C_6)$ alkyl which is unsubstituted or substituted with up to three substituents which can be the same or different and are selected from chlorine, bromine, fluorine, hydroxy and $(C_1-C_6)$ alkoxy.

2. A compound as claimed in Claim 1, wherein

Q is a group of the formula

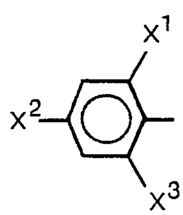

$X^1$ is a hydrogen atom, a chlorine atom, or a fluorine atom;
$X^2$ is a trifluoromethyl group;
$X^3$ is a hydrogen atom;
Y is a nitro group, a chlorine atom, or a bromine atom;
$R^1$ and $R^2$, independently of each other and in each ($CHR^1$) and ($CHR^2$) group, are a hydrogen atom or an unsubstituted or substituted ($C_1$—$C_6$) alkyl group;
A is —N($R^1$)—, —O—, —S—, —C(O)—, or —C(O)B—;
B is —O—, —N($R^4$)—, or a ($C_1$—$C_6$) alkylene group;
$R^4$ is a hydrogen atom or an unsubstituted or substituted ($C_1$—$C_6$) alkyl group;
Z is —$OR^5$ or —N($R^4$)$SO_2R^6$;
$R^5$ is an unsubstituted or substituted ($C_1$—$C_6$) alkyl group, a hydrogen atom, or an agronomically-acceptable alkaline earth metal, an alkali metal, or ammonium or substituted ammonium cation;
$R^6$ is unsubstituted ($C_1$—$C_6$) alkyl group;
m is 2 or 3; and
n is 1.

3. A compound as claimed in Claim 2, wherein:
A is —N(H)—, —N($CH_3$)—, —N($CH_2OCH_3$)—, —O—, —S—, —C(O)—, or —C(O)B—;
B is —O— or a ($C_1$—$C_6$) alkylene group; and
Z is $OR^5$.

4. A compound as claimed in Claim 3, wherein:
A is —N(H)—, —O—, —S—, —C(O)—, or —C(O)B—; and
B is —O—.

5. A compound as claimed in Claim 4, wherein $R^5$ is a hydrogen atom, an unsubstituted or substituted ($C_1$—$C_6$) alkyl group or a sodium, potassium, ammonium, or mono-, di-, tri- or tetraalkylammonium cation.

6. A compound as claimed in Claim 5, wherein:
$R^1$ and $R^2$, independently of each other and in each ($CHR^1$) and ($CHR^2$) group, are a hydrogen atom or a methyl group;
A is —O—;
$R^3$ is —C(O)Z;
$R^5$ is a methyl group;
m is 2 or 3; and
n is 1.

7. A compound as claimed in Claim 2, wherein $X^1$ is chlorine, $X^2$ is trifluoromethyl, $X^3$ is hydrogen, Y is nitro and R is —$CH_2CH_2OCH_2CO_2CH_3$, —$CH(CH_3)CH_2OCH_2CO_2CH_3$ or $CH_2CH(CH_3)OCH_2CO_2CH_3$.

8. A compound as claimed in Claim 5, wherein:
Y is a nitro group or a chlorine atom;
$R^1$ and $R^2$ are each a hydrogen atom;
A is —N(H)—, —C(O)— or —C(O)B—;
B is —O—;
$R^3$ is —C(O)Z;
Z is —$OR^5$;
$R^5$ is a methyl group;
m is 2 or 3; and
n is 1.

9. A compound as claimed in Claim 5, wherein:
Y is a nitro group or a chlorine atom;
$R^1$ and $R^2$, independently of each other and in each ($CHR^1$) and ($CHR^2$) group, are a hydrogen atom or a methyl group;
A is —S—;
Z is —$OR^5$;
$R^5$ is a methyl group;
m is 2 or 3; and
n is 1.

10. A herbicidal composition comprising a herbicidally-effective amount of a compound as claimed in any preceding claim and an agronomically-acceptable carrier or diluent.

11. A method of combating weeds which comprises applying to weed seedlings in a growth medium or the growth medium prior to the emergence of the weeds therefrom at least one compound as claimed in any one of Claims 1—9 in an amount sufficient to combat the growth of the weeds.

12. A method as claimed in Claim 11 wherein the growth medium contains seeds or growing plants capable of yielding an agronomic crop or said compound is applied prior to, or at the same time as, the planting of seeds or plants of an agronomic crop.

**Patentansprüche**

1. Ether mit zwei aromatischen Gruppen, die mit dem Ethersauerstoffatom verbunden sind und eine substituierte Aminogruppe aufweisen, die mit einem Phenylring verknüpft ist, dadurch gekennzeichnet, daß der Ether der Formel

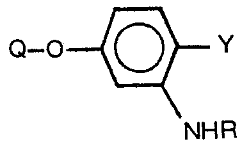

(I)

entspricht, worin

Q eine Gruppe der Formel

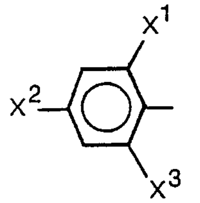

oder

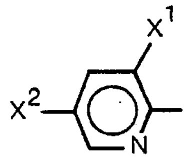

ist,

$X^1$ ein Wasserstoffatom, eine Cyanogruppe oder ein Halogenatom ist,

$X^2$ eine Halogenalkylgruppe oder ein Halogenatom ist,

$X^3$ ein Wasserstoffatom oder ein Halogenatom ist,

Y eine Nitrogruppe, eine Cyanogruppe oder ein Halogenatom ist,

R eine Gruppe der Formel

$$-(CHR^1)_m-A-(CHR^2)_n-R^3$$

ist,

$R^1$ und $R^2$ unabhängig voneinander und in jeder Gruppe ($CHR^1$) und ($CHR^2$) ein Wasserstoffatom, eine nichtsubstituierte oder substituierte ($C_1$—$C_6$)-Alkylgruppe, eine ($C_1$—$C_6$)-Alkoxygruppe oder eine ($C_1$—$C_6$)-Alkylthiogruppe sind, unter der Voraussetzung, daß $R^1$ und $R^2$ kein Alkyoxy oder Alkylthio in alpha-Stellung zu einem —NH-Anteil sind,

A —N($R^1$)—, —N(O)—, —O—, —S—, —S(O)—, —S(O)$_2$, —C(O)—, —C(O)B— oder —N(H)S(O)$_2$— ist,

B eine ($C_1$—$C_6$)-Alkylengruppe, —O—, —S— oder —N($R^4$)— ist;

$R^3$ eine Cyanogruppe oder eine Gruppe der Formel —C(O)Z ist, unter der Voraussetzung, daß dann, wenn $R^3$ Cyano ist, B eine ($C_1$—$C_6$)-Alkylengruppe oder —N($R^4$) ist;

$R^4$ ein Wasserstoffatom oder ein nichtsubstituierte oder substituierte ($C_1$—$C_6$)Alkylgruppe ist;

Z ein Wasserstoffatom, eine nichtsubstituierte oder substituierte ($C_1$—$C_6$)-Alkylgruppe, —N($R^4$)$_2$, —O$R^5$, —S$R^5$ oder —N($R^4$)SO$_2R^6$ ist, vorausgesetzt, daß dann, wenn Z Wasserstoff ist, A nicht —N(H)— oder —N(H)S(O)$_2$— ist, B nicht —N(H)— ist und $R^1$, $R^2$ oder $R^4$, falls mit einer Alkylgruppe substituiert, keine Amino- oder Mono-($C_1$—$C_6$)-Alkylaminogruppe sind,

$R^5$ ein Wasserstoffatom, eine nichtsubstituierte oder substituierte ($C_1$—$C_6$)-Alkylgruppe oder ein für landwirtschaftliche Zwecke verträgliches Kation ist,

$R^6$ eine nichtsubstituierte ($C_1$—$C_6$)-Alkylgruppe, eine Hydroxygruppe oder ein für landwirtschaftliche Zwecke verträgliches Salz, gebildet mit einer derartigen Gruppe, eine nichtsubstituierte oder substituierte Phenylgruppe mit bis zu 3 Substituenten, die gleich oder verschieden sein können und ausgewählt sind aus Chlor, Brom, Fluor, ($C_1$—$C_6$)-Alkoxy und ($C_1$—$C_6$)-Alkyl, wobei die Alkylgruppe nichtsubstituiert ist oder substituiert ist mit bis zu 3 Substituenten, die gleich oder verschieden sein können und ausgewählt sind aus Chlor, Brom und ($C_1$—$C_6$)-Alkoxy ist,

m eine ganze Zahl von 2 bis 10 ist und

n eine ganze Zahl von 1 bis 3 ist,

und wobei dann,

wenn $R^1$, $R^2$, $R^4$ oder $R^5$ eine substituierte Alkylgruppe sind, bis zu 3 Substituenten vorliegen können, die gleich oder verschieden sein können und ausgewählt sind aus Chlor, Brom, Fluor, Hydroxy, ($C_1$—$C_6$)-Alkoxy, ($C_1$—$C_6$)-Alkylthio, ($C_1$—$C_6$)-Alkylcarbonyl, ($C_1$—$C_6$)-Alkoxycarbonyl, Amino, Mono- oder Di-($C_1$—$C_6$)-Alkylamino, ($C_1$—$C_6$)-Alkylcarbonyloxy sowie nichtsubstituiertem oder substituiertem Phenyl, Phenoxy oder Phenylthio, wobei jede dieser aromatischen Gruppen bis zu 3 Substituenten besitzt, die gleich oder verschieden sein können und ausgewählt sind aus Chlor, Brom, Fluor, Hydroxy, ($C_1$—$C_6$)-Alkoxy, ($C_1$—$C_6$)-Alkylthio sowie ($C_1$—$C_6$)-Alkyl, das nichtsubstituiert oder substituiert ist mit bis zu 3 Substituenten, die gleich oder verschieden sein können und ausgewählt sind aus Chlor, Brom, Fluor, Hydroxy und ($C_1$—$C_6$)-Alkoxy.

2. Verbindung nach Anspruch 1, worin
Q eine Gruppe der Formel ist

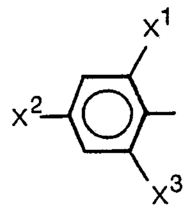

$X^1$ ein Wasserstoffatom, ein Chloratom oder ein Fluoratom ist,
$X^2$ eine Trifluormethylgruppe ist,
$X^3$ ein Wasserstoffatom ist,
Y eine Nitrogruppe, ein Chloratom oder ein Bromatom ist,
$R^1$ und $R^2$ unabhängig voneinander und in jeder Gruppe ($CHR^1$) und ($CHR^2$) ein Wasserstoffatom oder eine nichtsubstituierte oder substituierte ($C_1$—$C_6$)-Alkylgruppe sind,
A —N($R^1$)—, —O—, —S—, —C(O)— oder —C(O)B— ist,
B —O—, —N($R^4$)— oder eine ($C_1$—$C_6$)-Alkylengruppe ist,
$R^4$ ein Wasserstoffatom oder eine nichtsubstituierte oder substituierte ($C_1$—$C_6$)-Alkylgruppe ist,
Z —$OR^5$ oder —N($R^4$)$SO_2R^6$ ist,
$R^5$ eine nichtsubstituierte oder substituierte ($C_1$—$C_6$)-Alkylgruppe, ein Wasserstoffatom oder ein für landwirtschaftliche Zwecke verträgliches Erdalkalimetall-, Alkalimetall- oder Ammonium- oder substituiertes Ammonium-Kation ist,
$R^6$ eine nichtsubstituierte ($C_1$—$C_6$)-Alkylgruppe ist,
m 2 oder 3 ist und
n 1 ist.

3. Verbindung nach Anspruch 2, worin
A —N(H)—, —N($CH_3$)—, —N($CH_2OCH_3$)—, —O—, —S—, —C(O)— oder —C(O)B ist,
B —O— oder eine ($C_1$—$C_6$)-Alkylengruppe ist und
Z $OR^5$ ist.

4. Verbindung nach Anspruch 3, worin
A —N(H)—, —O—, —S—, —C(O)— oder —C(O)B— ist und
B —O— ist.

5. Verbindung nach Anspruch 4, worin $R^5$ ein Wasserstoffatom, eine nichtsubstituierte oder substituierte ($C_1$—$C_6$)-Alkylgruppe oder ein Natrium-, Kalium-, Ammonium- oder Mono-, Di-, Tri- oder Tetraalkylammoniumkation ist.

6. Verbindung nach Anspruch 5, worin
$R^1$ und $R^2$ unabhängig voneinander in jeder Gruppe ($CHR^1$) und ($CHR^2$) ein Wasserstoffatom oder eine Methylgruppe sind,
A —O— ist,
$R^3$ —C(O)Z ist,
$R^5$ eine Methylgruppe ist,
m 2 oder 3 ist und
n 1 ist.

7. Verbindung nach Anspruch 2, worin $X^1$ Chlor ist, $X^2$ Trifluormethyl ist, $X^3$ Wasserstoff ist, Y Nitro ist und R —$CH_2CH_2OCH_2CO_2CH_3$, —$CH(CH_3)CH_2OCH_2$— $CO_2CH_3$ oder $CH_2CH(CH_3)OCH_2CO_2CH_3$ ist.

8. Verbindung nach Anspruch 5, worin
Y eine Nitrogruppe oder ein Chloratom ist,
$R^1$ und $R^2$ jeweils ein Wasserstoffatom sind,
A —N(H)—, —C(O)— oder —C(O)B— ist,
B —O— ist,
$R^3$ —C(O)Z ist,
Z —$OR^5$ ist,
$R^5$ eine Methylgruppe ist,
m 2 oder 3 ist und
n 1 ist.

9. Verbindung nach Anspruch 5, worin
Y eine Nitrogruppe oder ein Chloratom ist,
$R^1$ und $R^2$ unabhängig voneinander und in jeder Gruppe ($CHR^1$) und ($CHR^2$) ein Wasserstoffatom oder eine Methylgruppe sind,
A —S— ist,
Z —$OR^5$ ist,
$R^5$ eine Methylgruppe ist,
m 2 oder 3 ist und
n 1 ist.

10. Herbizides Mittel aus einer herbizid wirksamen Menge einer Verbindung gemäß einem der vorhergehenden Ansprüche und einem für landwirtschaftliche Zwecke verträglichen Träger oder Verdünnungsmittel

11. Verfahren zur Bekämpfung von Unkräutern, welches darin besteht, daß auf Unkrautsämlinge in einem Wachstumsmedium oder auf das Wachstumsmedium vor dem Auflaufen der Unkräuter daraus wenigstens eine Verbindung gemäß einem der Ansprüche 1 bis 9 in einer Menge aufgebracht wird, die dazu ausreicht, das Wachstum der Unkräuter zu bekämpfen.

12. Verfahren nach Anspruch 11, wobei das Wachstumsmedium Sämlinge oder wachsende Pflanzen enthält, die dazu in der Lage sind, eine landwirtschaftliche Frucht zu ergeben oder die Verbindung vor oder gleichzeitig mit dem Pflanzen von Sämlingen oder Pflanzen einer landwirtschaftlichen Frucht appliziert wird.

**Revendications**

1. Un éther ayant deux groupes aromatiques fixés à l'atome d'oxygène de type éther et ayant un groupe amino substitué fixé à un cycle phényle, cet éther étant caractérisé en ce qu'il répond à la formule:

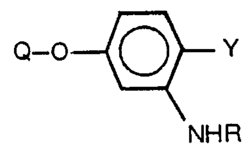

(I)

dans laquelle

Q est un groupe de formule

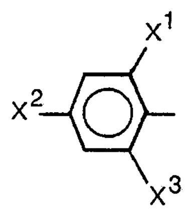

ou

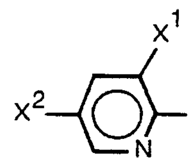

$X^1$ est un atome d'hydrogène, un groupe cyano ou un atome d'halogène;

$X^2$ est un groupe halogénoalcoyle, ou un atome d'halogène;

$X^3$ est un atome d'hydrogène ou un atome d'halogène;

Y est un groupe nitro, un groupe cyano ou un atome d'halogène;

R est un groupe de formule

$$-(CHR^1)_m-A-(CHR^2)_n-R^3$$

$R^1$ et $R^2$, indépendamment l'un de l'autre et dans chaque groupe ($CHR^1$) et ($CHR^2$), sont un atome d'hydrogène; un groupe alcoyle ($C_1$—$C_6$) substitué ou non substitué; un groupe alcoxy ($C_1$—$C_6$); ou un groupe alcoylthio ($C_1$—$C_6$); sous réserve que $R^1$ et $R^2$ ne peuvent pas être un alcoxy ou un alcoylthio en α d'un fragment —NH—;

A est $—N(R^1)—$, —N(O)—, —O—, —S—, —S(O)—, $—S(O)_2—$, —C(O)—, —C(O)B—, ou $—N(H)S(O)_2—$;

B est un groupe alcoylène ($C_1$—$C_6$), —O—, —S— ou $—N(R^4)—$;

$R^3$ est un groupe cyano ou un groupe de formule —C(O)Z, sous réserve que lorsque $R^3$ est un cyano, B est un groupe alcoylène ($C_1$—$C_6$) ou $—N(R^4)—$;

$R^4$ est un atome d'hydrogène ou un groupe alcoyle ($C_1$—$C_6$) non substitué ou substitué;

Z est un atome d'hydrogène; un groupe alcoyle ($C_1$—$C_6$) non substitué ou substitué; $—N(R^4)_2$; $—OR^5$; $—SR^5$; ou $—N(R^4)SO_2R^6$, sous réserve que lorsque Z est un hydrogène, A n'est pas —N(H)— ni $—N(H)S(O)_2—$;

B n'est pas —N(H)— et $R^1$, $R^2$ ou $R^4$, lorsqu'ils sont des groupes alcoyles substitués, ne comprennent pas de groupe amino ou monoalcoyl($C_1$—$C_6$) amino;

$R^5$ est un atome d'hydrogène; un groupe alcoyle ($C_1$—$C_6$) non substitué ou substitué ou un cation acceptable en agronomie;

$R^6$ est un groupe alcoyle ($C_1$—$C_6$) non substitué, un groupe hydroxy ou un sel convenant en agronomie formé avec un tel groupe, un groupe phényle non substitué ou substitué avec jusqu'à trois substituants qui peuvent être semblables ou différents et sont choisis parmi le chlore, le brome, le fluor, un alcoxy ($C_1$—$C_6$) et un alcoyle ($C_1$—$C_6$) lequel groupe alcoyle est non substitué ou substitué par jusqu'à trois substituants qui peuvent être semblables ou différents et sont choisis parmi le chlore, le brome et un alcoxy ($C_1$—$C_6$);

m est un entier de 2 à 10; et

n est un entier de 1 à 3;

et dans laquelle

lorsque $R^1$, $R^2$, $R^4$ ou $R^5$ est un groupe alcoyle substitué, il peut comporter jusqu'à trois substituants qui peuvent être semblables ou différents et sont choisis parmi le chlore, le brome, le fluor, un hydroxy, un alcoxy ($C_1$—$C_6$), un alcoylthio ($C_1$—$C_6$), un alcoyl($C_1$—$C_6$) carbonyle, un alcoxy($C_1$—$C_6$) carbonyle, un amino, un mono- ou di-alcoyl($C_1$—$C_6$) amino, un alcoyl($C_1$—$C_6$) carbonyloxy et un phényle, un phénoxy ou un phénylthio non substitué ou substitué, chacun de ces groupes aromatiques ayant jusqu'à trois substituants qui peuvent être semblables ou différents et sont choisis parmie le chlore, le brome, le fluor, un hydroxy, un alcoxy($C_1$—$C_6$), un alcoylthio($C_1$—$C_6$) et un alcoyle ($C_1$—$C_6$) qui est non substitué ou est substitué par jusqu'à trois substituants qui peuvent être semblables ou différents et sont choisis parmi le chlore, le brome, le fluor, un hydroxy et un alcoxy ($C_1$—$C_6$).

2. Un composé selon la revendication 1 dans lequel Q est un groupe de formule

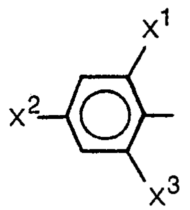

$X^1$ est un atome d'hydrogène, un atome de chlore ou un atome de fluor;
$X^2$ est un groupe trifluorométhyle;
$X^3$ est un atome d'hydrogène;
Y est un groupe nitro, un atome de chlore ou un atome de brome;
$R^1$ et $R^2$, indépendamment l'un de l'autre et dans chaque groupe ($CHR^1$) et ($CHR^2$), sont un atome d'hydrogène ou un groupe alcoyle ($C_1$—$C_6$) non substitué ou substitué;
A est —N($R^1$)—, —O—, —S—, —C(O)— ou —C(O)B—;
B est —O—, —N($R^4$)— ou un groupe alcoylène($C_1$—$C_6$);
$R^4$ est un atome d'hydrogène ou un groupe alcoyle ($C_1$—$C_6$) non substitué ou substitué;
Z est —$OR^5$ ou —N($R^4$)$SO_2R^6$;
R est un groupe alcoyle ($C_1$—$C_6$) non substitué ou substitué, un atome d'hydrogène ou un cation convenant en agronomie de métal alcalino-terreux, de métal alcalin ou ammonium ou ammonium substitué;
$R^6$ est un groupe alcoyle ($C_1$—$C_6$) non substitué;
m est 2 ou 3; et
n est 1.

3. Un composé comme revendiqué dans la revendication 2 dans lequel:
A est —N(H)—, —N($CH_3$)—, —N($CH_2OCH_3$)—, —O—, —S—, —C(O)— ou —C(O)B—;
B est —O— ou un groupe alcoylène ($C_1$—$C_6$); et
Z est $OR^5$.

4. Un composé comme revendiqué dans la revendication 3 dans lequel:
A est —N(H)—, —O—, —S—, —C(O)— ou —C(O)B—; et
B est —O—.

5. Un composé selon la revendication 4 dans lequel $R^5$ est un atome d'hydrogène, un groupe alcoyle ($C_1$—$C_6$) non substitué ou substitué ou un cation de sodium, de potassium, ammonium ou mono-, di-, tri- ou tétraalcoylammonium.

6. Un composé comme revendiqué dans la revendication 5 dans lequel:
$R^1$ et $R^2$, indépendamment l'un de l'autre et dans chaque groupe ($CHR^1$) et ($CHR^2$), sont un atome d'hydrogène ou un groupe méthyle;
A est —O—;
$R^3$ est —C(O)Z;
$R^5$ est un groupe méthyle;
m est 2 ou 3; et
n est 1.

7. Un composé comme revendiqué dans la revendication 2 dans lequel $X^1$ est un chlore, $X^2$ est un trifluorométhyle, $X^3$ est un hydrogène, Y est un nitro et R est —$CH_2CH_2OCH_2CO_2CH_3$, —$CH(CH_3)CH_2OCH_2CO_2CH_3$ ou $CH_2CH(CH_3)OCH_2CO_2CH_3$.

8. Un composé comme revendiqué dans la revendication 5 dans lequel:
Y est un groupe nitro ou un atome de chlore;
$R^1$ et $R^2$ sont chacun un atome d'hydrogène;
A est —N(H)—, —C(O)— ou —C(O)B—;
B est —O—;
$R^3$ est —C(O)Z;
Z est —$OR^5$;
$R^5$ est un groupe méthyle;
m est 2 ou 3; et

n est 1.

9. Un composé comme revendiqué dans la revendication 5 dans lequel:

Y est un groupe nitro ou un atome de chlore;

$R^1$ et $R^2$, indépendamment l'un de l'autre et dans chaque groupe ($CHR^1$) et ($CHR^2$), sont un atome d'hydrogène ou un groupe méthyle;

A est —S—;

Z est —$OR^5$;

$R^5$ est un groupe méthyle;

m est 2 ou 3; et

n est 1.

10. Une composition herbicide comprenant une quantité herbicide efficace d'un composé comme revendiqué dans l'une quelconque des revendications précédentes et un support ou diluant convenant en agronomie.

11. Un procédé de lutte contre les mauvaises herbes qui comprend l'application aux pousses des mauvaises herbes dans un milieu de culture ou au milieu de culture avant le levée des mauvaises herbes, d'au moins un composé comme revendiqué dans l'une quelconque des revendications 1 à 9, en une quantité suffisante pour lutter contre la croissance des mauvaises herbes.

12. Un procédé comme revendiqué dans la revendication 11 dans lequel le milieu de culture contient des semences ou des plantes en croissance capables de produire une culture agronomique ou ledit composé est appliqué avant, ou simultanément à, la mise en terre des semences ou des plantes d'une culture agronomique.